# EUROPÄISCHE PATENTSCHRIFT

(11) **EP 2 548 613 B1**
(45) Veröffentlichungstag und Bekanntmachung des Hinweises auf die Patenterteilung: **04.03.2015**
(21) Anmeldenummer: 12174540.0
(22) Anmeldetag: 02.07.2012
(51) Int. Cl.: A61N 1/39, A61N 1/05

(54) **Implantierbare Defibrillationsanordnung und Elektrodenleitung**
Implantable defibrillation arrangement and electrode lead
Système de défibrillation implantable et dérivation d'électrodes

(30) Priorität: 21.07.2011 US 201161510082 P
(43) Veröffentlichungstag der Anmeldung: 23.01.2013
(73) Patentinhaber: BIOTRONIK SE & Co. KG, 12359 Berlin (DE)
(72) Erfinder: Dörr, Thomas, 12437 Berlin (DE)
(74) Vertreter: Lindner-Vogt, Karin L.

(56) Entgegenhaltungen:
- EP-A2- 2 143 466
- WO-A1-00/20071
- US-A- 5 325 870
- US-A- 5 336 253
- US-A- 5 545 183

## Beschreibung

Die Erfindung betrifft eine implantierbare Defibrillationsanordnung, umfassend ein Defibrillationsgerät mit einer Abfühlkomponente und einer Defibrillationskomponente, sowie eine Elektrodenleitung mit einem Leitungskörper, einem Stecker, einer Abfühlelektrode zum Abfühlen von Herzaktionspotenzialen mit einer ersten Elektrodenzuleitung und einer Defibrillationselektrode zur Übertragung von Schockimpulsen an Herzgewebe mit einer zweiten Elektrodenzuleitung.

Implantierbare Defibrillatoren oder Kardioverter (ICD) sind seit langem bekannt und im klinischen Einsatz und seit Jahrzehnten Gegenstand permanenter technischer Weiterentwicklung. In noch stärkerem Maße gilt dies für implantierbare Herzschrittmacher und die zugehörigen Elektrodenleitungen. Als spezielle Geräteklasse haben sich auch kombinierte Herzstimulations- und Defibrillationsanordnungen, einschließlich der speziell hierfür entwickelten Elektrodenleitungen (ICD-Elektroden), auf dem Gerätemarkt und in der klinischen Praxis etabliert. Derartige Kombinationsgeräte werden als Herzschrittmacher zur kardialen Resynchronisations-Therapie mit Defibrillator, abgekürzt als CRT-D-Geräte, bezeichnet.

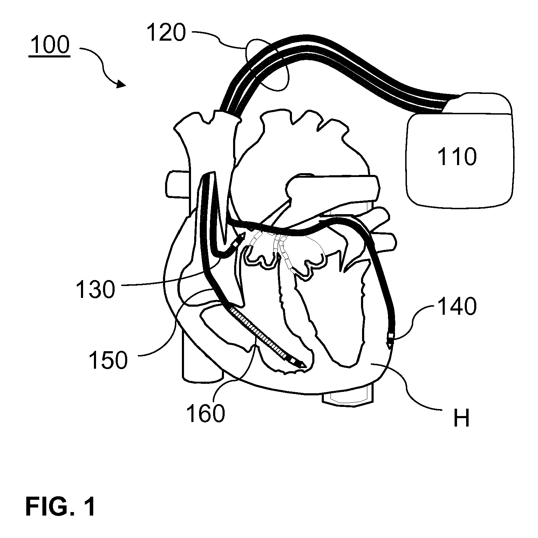

Fig. 1 zeigt eine schematische Darstellung einer solchen Anordnung 100 mit in das Herz H eines Patienten verlegten Elektroden. Demnach ist ein Herzstimulations- und Defibrillationsgerät 110 über eine Elektrodenleitung 120 mit dem Herzen H verbunden, die drei Leitungszweige bzw. Elektrodenzuleitungen 130, 140 und 150 umfasst. Alle Leitungszweige tragen an oder nahe ihrem distalen Ende (nicht einzeln bezeichnete) Abfühl- bzw. Stimulationselektroden, und der Leitungszweig 150 trägt zudem eine langgestreckte Defibrillationselektrode 160. In der gezeigten Anordnung ist der Leitungszweig 130 im rechten Atrium und der Leitungszweig 140 im linken Ventrikel des Herzens H verlegt, und der die Defibrillationselektrode 160 tragende Leitungszweig 150 ist im rechten Ventrikel (RV) verlegt.

Bei der Schockabgabe wird hier nur die Schockwendel der RV-Elektrode auf ein Hochspannungspotential geschaltet, während dessen das bipolare Wahrnehmungs- und Stimulationssystem auf dem Ausgangspotential verbleiben. Daher müssen die Zuleitungen innerhalb der Elektrodenleitung 120 mit hochspannungsfesten Isolationsabständen realisiert werden.

Aus den Schriften US5,545,183 und US5,336,253 sind Elektrodenleitungen und implantierbare Defibrillatoren mit Elektrodenleitungen bekannt, die ein Schalten der Elektroden ermöglichen.

In allen derzeit bekannten ICD-Elektroden müssen innerhalb der Elektrodenleitung Isolationsabstände für Hochspannungen eingehalten werden, was eine weitere Reduktion des Elektrodendurchmessers limitiert bzw. auch die Integration einer Schockwendel auf eine CS-Elektrode verhindert.

Hieraus ergibt sich als Aufgabe der Erfindung die Bereitstellung einer verbesserten implantierbaren Defibrillationsanordnung, die insbesondere eine leichter verlegbare und flexibel in unterschiedlichen Anwendungssituationen einsetzbare Elektrodenleitung umfasst und somit insgesamt breiter und einfacher klinisch einsetzbar ist.

Diese Aufgabe wird durch eine Defibrillationsanordnung mit den Merkmalen des Anspruchs 1 gelöst. Zweckmäßige Ausgestaltungen des Erfindungsgedankens sind Gegenstand der jeweiligen abhängigen Ansprüche.

Die Erfindung zieht darauf ab, in einer Anordnung der genannten Art eine wesentliche Reduzierung des Durchmessers und Erhöhung der Flexibilität der Elektrodenleitung dadurch zu erreichen, dass die erforderlichen Isolationswiderstände innerhalb der Leitung wesentlich reduziert werden. Hierzu schließt die Erfindung den weiteren Gedanken ein, im kritischen Einsatzfall der Anordnung, nämlich bei Abgabe eines Defibrillations- bzw. Schockimpulses mit hoher Spannung, die auftretenden Potentialunterschiede zwischen der Defibrillationselektrode und der Abfühlelektrode oder den Abfühlelektroden wesentlich reduziert. Schließlich gehört zur Erfindung der Gedanke, diese wesentliche Reduzierung durch eine Schalteinheit zum Schalten der oder jeder Abfühlelektrode auf das Potential der Defibrillationselektrode im Ansprechen auf die Ausgabe eines Defibrillationsschocks zu bewirken.

Die Erfindung ermöglicht somit die Realisierung von Elektrodenleitungen für ICD- bzw. CRT-D-Anordnungen mit wesentlich geringerem Durchmesser und geringerer Steifigkeit und trägt somit zur Realisierung von ICT- bzw. CRT-D-Systemen mit deutlich verbesserten Gebrauchseigenschaften bei.

Erfindungsgemäß ist die erwähnte Schalteinheit zum Schalten der Abfühlelektrode(n) auf das Potential der Defibrillationselektrode im Stecker der Elektrodenleitung angeordnet.

Die Schalteinheit weist ein spannungsabhängig selbsttätig wirkendes Schaltelement auf, insbesondere einen Varistor auf, etwa einen Varistor aus Sinterkeramik vom SiC- oder ZnO-Typ.

In einer Ausführung ist die vorgeschlagene Anordnung als Herzstimulations- und Defibrillationsanordnung (CRT-D-Gerät) ausgestaltet, wobei das Defibrillationsgerät eine Stimulationskomponente umfasst und die Elektrodenleitung eine Stimulationselektrode zur Übertragung von Stimulationsimpulsen an Herzgewebe auf der zweiten Elektrodenzuleitung oder mit einer dritten Elektrodenzuleitung aufweist und die Schalteinheit zum Schalten auch der Stimulationselektrode auf das Potential der Defibrillationselektrode ausgebildet ist.

Wie weiter oben bereits angemerkt, zeichnet sich eine zum Einsatz in der oben beschriebenen Defibrillationsanordnung geeignete Elektrodenleitung dadurch aus, dass die Schalteinheit ein spannungsabhängig selbsttätig wirkendes Schaltelement aufweist. Speziell in der Ausgestaltung als CRT-D-Gerät mit einer Stimulationselektrode zum Anlegen von Stimulationsimpulsen an Herzgewebe mit einer dritten Elektrodenzuleitung, ist diese bezüglich der zweiten Elektrodenzuleitung im Leitungskörper mit einem Abstand und einer gegenseitigen Isolierung verlegt, die unterhalb der Anforderungen an die Hochspannungsfestigkeit bei Übertragung üblicher Defibrillationsspannungen über die Elektrodenleitung liegen.

Bezüglich weiterer spezieller Ausführungen der Elektrodenleitung kann auf vorstehende Erläuterungen verwiesen werden.

Vorteile und Zweckmäßigkeiten der Erfindung ergeben sich im Übrigen aus der nachfolgenden Beschreibungen von Ausführungsbeispielen anhand der Figuren. Von diesen zeigen:
- Fig. 1: eine schematische Darstellung einer herkömmlichen Herzstimulations- und Defibrillationsanordnung,
- Fig. 2: ein Blockschaltbild einer Ausführungsform der erfindungsgemäßen Defibrillationsanordnung und
- Fig. 3: ein Blockschaltbild einer weiteren Ausführungsform der erfindungsgemäßen Defibrillationsanordnung.

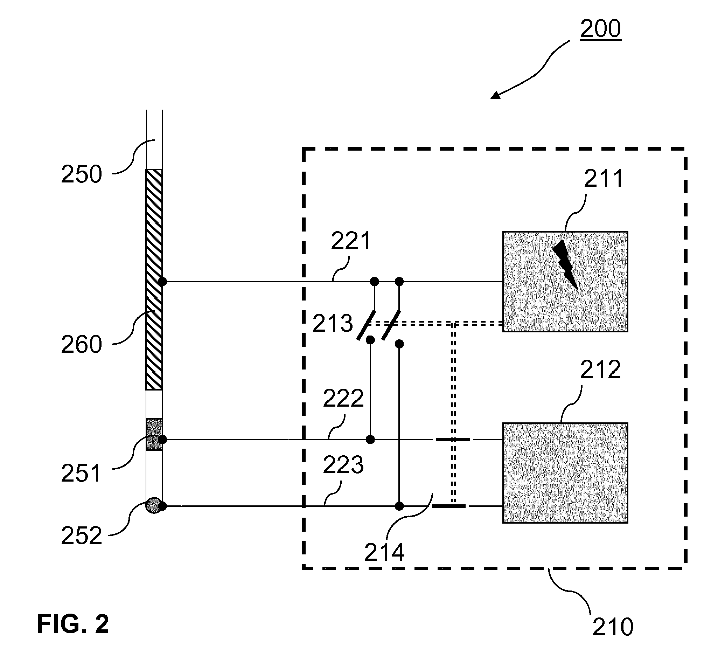

Fig. 2 zeigt eine Herzstimulations- und Defibrillationsanordnung 200, die aus einem Stimulations- und Defibrillationsgerät 210 und einer Elektrodenleitung bzw. einem Elektrodenleitungszweig 250 gebildet ist, wobei der Aufbau des Letzteren grundsätzlich dem des Leitungszweigs 150 bei der in Fig. 1 gezeigten Anordnung entspricht. Die Elektrodenleitung 250 ist als bipolares System zur Wahrnehmung bzw. zum Abfühlen von Herzaktionspotentialen und zur Stimulation des Ventrikels ausgeführt und weist entsprechende Elektroden 251 und 252 auf. Zusätzlich trägt sie eine Schockwendel 260 als Defibrillationselektrode. Gegenelektrode für die Defibrillation ist ein (nicht gesondert bezeichnetes) Gehäuse des Stimulations- und Defibrillationsgerätes 210. Das Gerät 210 umfasst in an sich bekannter Weise eine Defibrillationskomponente 211 und eine Abfühl- und Stimulationskomponente 212.

Um die Isolationsabstände der Elektrodenzuleitungen 222, 223 der Abfühl- und Stimulationselektroden 251, 252 einerseits und der Zuleitung 221 der Schockelektrode 260 andererseits reduzieren zu können, ist neben diesen Gerätekomponenten eine Schalteinheit 213 vorgesehen, die bei Abgabe eines Defibrillationsschocks die Abfühl- und Stimulationselektroden 251, 252 auf das Potential der Defibrillationselektrode 260 schaltet. Als Ansteuereinheit für die Schalteinheit 213 dient hier ein ohnehin vorhandener Hochspannungsschutzschalter 214 der Abfühl- und Stimulationskomponente 212, der seinerseits über ein Steuersignal von der Defibrillationskomponente 211 gesteuert wird und der Schalteinheit 213 ein geeignetes Steuerpotential bereit stellt.

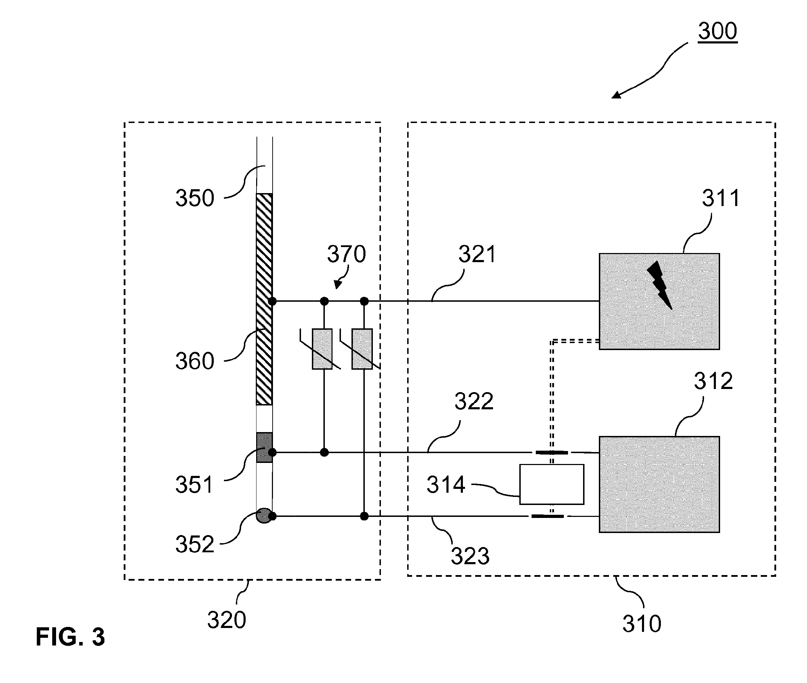

Fig. 3 zeigt eine modifizierte Ausführung dieser Anordnung, wobei gleiche oder einander funktional entsprechende Teile mit an Fig. 2 angelehnten Bezugsziffern bezeichnet sind und hier nicht nochmals erläutert werden. Der wesentliche Unterschied gegenüber der in Fig. 2 gezeigten Anordnung besteht darin, dass die Umschaltung des Potentials der Abfühl- und Stimulationselektroden 351, 352 auf das Potential der Defibrillationselektrode 363 bei Ausgabe eines Defibrillationsschocks hier durch eine in die Elektrodenleitung 323 integrierte Schalteinheit 370 bewirkt wird, statt durch eine im Stimulations- und Defibrillationsgerät eingebaute Komponente. 314 bezeichnet einen Hochspannungsschutzschalter analog zu 214.

Bei der gezeigten Ausführung umfasst die selbsttätig wirkende (also ohne Ansteuersignal aus dem CRT-D-Gerät auskommende) Schalteinheit 370 zwei Varistoren 371, 372, die zwischen die Elektrodenzuleitung 321, die die Defibrillationskomponente 311 und der Defibrillationselektrode 360 verbindet, und eine der beiden weiteren Zuleitungen 322, 323 geschaltet sind, welche die Abfühl- und Stimulationskomponente 312 mit den Abfühl- und Stimulationselektroden 351 und 352 verbinden.

Die in Fig. 3 gezeigte Anordnung kann in vorteilhafter Weise auch aus einem an sich bekannten, also nicht erfindungsgemäß ausgestalteten, Stimulations- und Defibrillationsgerät und einer speziell gestalteten Elektrodenleitung gebildet werden, die die erforderlichen (selbsttätig wirkenden) Schaltelemente enthält.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare Defibrillationsanordnung (100, 300), umfassend ein Defibrillationsgerät (110, 310) mit einer Abfühlkomponente (312) und einer Defibrillationskomponente (311) sowie eine Elektrodenleitung (150, 350) mit einem Leitungskörper, einem Stecker, einer Abfühlelektrode (351, 352) zum Abfühlen von Herzaktionspotenzialen mit einer ersten Elektrodenzuleitung (323) und einer Defibrillationselektrode (160,360) zur Übertragung von Schockimpulsen an Herzgewebe mit einer zweiten Elektrodenzuleitung (321),
wobei eine Schalteinheit zum Schalten der Abfühlelektrode auf das Potential der Defibrillationselektrode im Ansprechen auf die Ausgabe eines Defibrillationsschocks durch die Defibrillationskomponente vorgesehen ist,
wobei die erste und zweite Elektrodenzuleitung im Leitungskörper mit einem Abstand und einer gegenseitigen Isolierung verlegt sind, die unterhalb der Anforderungen an die Hochspannungsfestigkeit bei Übertragung üblicher Defibrillationsspannungen über die Elektrodenleitung liegen, wobei
jeweils ein spannungsabhängig selbsttätig wirkendes Schaltelement zwischen die zweite Elektrodenzuleitung (321) und die erste (323) und eine optional vorgesehene dritte Elektrodenzuleitung (322) geschaltet ist, **dadurch gekennzeichnet, dass** das oder jedes spannungsabhängige Schaltelement im Stecker angeordnet ist.

2. Defibrillationsanordnung nach Anspruch 1, wobei das Defibrillationsgerät eine Stimulationskomponente umfasst und die Elektrodenleitung eine Stimulationselektrode zur Übertragung von Stimulationsimpulsen an Herzgewebe über die zweite Elektrodenzuleitung oder mit der dritten Elektrodenzuleitung aufweist und die Schalteinheit zum Schalten auch der Stimulationselektrode auf das Potential der Defibrillationselektrode ausgebildet ist.

3. Defibrillationsanordnung nach Anspruch 1, mit einer Stimulationselektrode zum Anlegen von Stimulationsimpulsen an Herzgewebe mit der dritten Elektrodenzuleitung, die bezüglich der zweiten Elektrodenzuleitung im Leitungskörper mit einem Abstand und einer gegenseitigen Isolierung verlegt ist, die unterhalb der Anforderungen an die Hochspannungsfestigkeit bei Übertragung üblicher Defibrillationsspannungen über die Elektrodenleitung liegen.

4. Defibrillationsanordnung nach Anspruch 1, wobei die oder jede Schalteinheit einen Varistor aufweist.

## Claims

1. An implantable defibrillation assembly (100, 300), comprising a defibrillation device (110, 310) having a sensing component (312) and a defibrillation component (311), and an electrode lead (150, 350) having a lead body, a connector, a sensing electrode (351, 352) for sensing cardiac action potentials using a first electrode feed line (323) and a defibrillation electrode (160, 360) for transmitting shock pulses to heart tissue using a second electrode feed line (321),
wherein a switching unit for switching the sensing electrode to the potential of the defibrillation electrode during the response to the delivery of a defibrillation shock by the defibrillation component is provided,
wherein the first and second electrode feed lines are installed in the lead body with reciprocal insulation and at a distance below the requirements in regard to high voltage resistance for the transmission of common defibrillation voltages via the electrode lead,
wherein in each case a switching element acting automatically as a function of the voltage is connected between the second electrode feed line (321) and the first (323), and an optionally provided third electrode feed line (322),
**characterized in that** the or each voltage-dependent switching element is disposed in the connector.

2. The defibrillation assembly according to claim 1,
wherein the defibrillation device comprises a stimulation component, and the electrode lead comprises a stimulation electrode for transmitting stimulation pulses to heart tissue via the second electrode feed line or using the third electrode feed line, and the switching unit is designed to also switch the stimulation electrode to the potential of the defibrillation electrode.

3. The defibrillation assembly according to claim 1, comprising a stimulation electrode for applying stimulation pulses to heart tissue using the third electrode feed line, which with respect to the second electrode feed line is installed in the lead body with reciprocal insulation and at a distance below the requirements in regard to high voltage resistance for the transmission of common defibrillation voltages via the electrode lead.

4. The defibrillation assembly according to claim 1, wherein the or each switching unit comprises a varistor.

## Revendications

1. Ensemble de défibrillation implantable (100, 300), comprenant un appareil de défibrillation (110, 310) avec un composant de détection (312) et un composant de défibrillation (311), ainsi qu'une ligne d'électrode (150, 350) avec un corps de ligne, un connecteur, une électrode de détection (351, 352) pour la détection de potentiels d'activité cardiaque avec une première ligne d'alimentation d'électrode (323) et une électrode de défibrillation (160, 360) pour la transmission d'impulsions de choc à un tissu cardiaque avec une deuxième ligne d'alimentation d'électrode (321),
dans lequel il est prévu une unité de commutation pour commuter l'électrode de détection sur le potentiel de l'électrode de défibrillation en réponse à la délivrance d'un choc de défibrillation par le composant de défibrillation,
dans lequel les première et deuxième lignes d'alimentation d'électrode sont posées dans le corps de ligne avec un espacement et une isolation réciproque, qui sont en dessous des exigences concernant la tenue en haute tension lors de la transmission de tensions de défibrillation courantes par la ligne d'électrode, sachant que un élément de commutation automatique dépendant de la tension est respectivement raccordé entre la deuxième ligne d'alimentation d'électrode (321) et la première (323) et une troisième ligne d'alimentation d'électrode (322) prévue en option, **caractérisé en ce que** l'élément ou chaque élément de commutation dépendant de la tension est agencé dans le connecteur.

2. Ensemble de défibrillation selon la revendication 1, dans lequel l'appareil de défibrillation comprend un composant de stimulation et la ligne d'électrode comporte une électrode de stimulation destinée à transmettre des impulsions de stimulation à des tissus cardiaques par le biais de la deuxième ligne d'alimentation d'électrode ou avec la troisième ligne d'alimentation d'électrode, et l'unité de commutation est conçue pour commuter également l'électrode de stimulation sur le potentiel de l'électrode de défibrillation.

3. Ensemble de défibrillation selon la revendication 1, avec une électrode de stimulation destinée à appliquer des impulsions de stimulation à des tissus cardiaques avec la troisième ligne d'alimentation d'électrode, laquelle est posée dans le corps de ligne avec un espacement et une isolation réciproque par rapport à la deuxième ligne d'alimentation d'électrode, qui se trouvent en dessous des exigences concernant la tenue en haute tension lors de la transmission de tensions de défibrillation courantes par le biais de la ligne d'électrode.

4. Ensemble de défibrillation selon la revendication 1, dans lequel l'unité ou chaque unité de commutation comporte un varistor.
